**Europäisches Patentamt**

**European Patent Office**

**Office européen des brevets**

(19)

(11) Veröffentlichungsnummer: **0 178 483**
**B1**

(12) **EUROPÄISCHE PATENTSCHRIFT**

(45) Veröffentlichungstag der Patentschrift:
**19.04.89**

(51) Int. Cl.⁴: **A 61 F 2/52**

(21) Anmeldenummer: **85111855.4**

(22) Anmeldetag: **19.09.85**

(54) **Prothese als Ersatz für eine amputierte Brust.**

(30) Priorität: **13.10.84 DE 3437591**
**09.11.84 DE 3440960**

(43) Veröffentlichungstag der Anmeldung:
**23.04.86 Patentblatt 86/17**

(45) Bekanntmachung des Hinweises auf die Patenterteilung:
**19.04.89 Patentblatt 89/16**

(84) Benannte Vertragsstaaten:
**AT BE CH FR GB IT LI LU NL SE**

(56) Entgegenhaltungen:
**WO-A-82/00583**
**US-A-3 811 133**
**US-A-3 852 833**
**US-A-4 507 810**

(73) Patentinhaber: **S + G IMPLANTS GMBH,
Grapengiesserstrasse 21, D-2400 Lübeck (DE)**

(72) Erfinder: **Grundei, Hans, Gärtnergasse 4, D-2400
Lübeck (DE)**

(74) Vertreter: **Wilcken, Thomas, Dipl.- Ing.,
Musterbahn 1, D-2400 Lübeck (DE)**

## Beschreibung

Die Erfindung bezieht sich auf eine Prothese als Ersatz für eine amputierte Brust, bestehend aus einer der Brustform nachgebildeten Hülle aus elastischem Material mit einer Füllung aus einer träge fließenden Masse.

Bekannte Prothesen der vorerwähnten Art, die im allgemeinen durch einen Büstenhalter abgestützt und dem Brustkorb angepaßt werden, haben den Vorteil, daß sich die Prothese durch die träge fließende Masse, insbesondere gelartiger Silikon-Kautschuk, und die elastische Hülle bei Körperbewegungen wie eine normale Brust schwingend bewegen kann. Die Prothese ist durch die Hüllenfüllung aber verhältnismäßig schwer und wird daher von den Frauen als unangenehm empfunden.

Die Aufgabe der Erfindung besteht darin, die bekannten Prothesen so zu verbessern, daß ihr Gewicht wesentlich herabgesetzt wird, daß aber die schwingenden Bewegungen der Prothese wie bei der natürlichen Brust weiter gewährleistet werden.

Aus der US-A-3 852 833 ist bereits eine Brustprothese mit zwei Kammern bekannt, bei der die dem Brustkorb zugekehrte Kammer mit Luft und die äußere Kammer mit Flüssigkeit gefüllt ist. Ferner ist aus der US-A-3 811 133 bekannt, die Hülle einer Brustprothese mit Fasermaterial und einem Gewicht zu füllen.

Die vorstehend genannte Aufgabe wird nach der Erfindung dadurch gelöst, daß bei der eingangs erwähnten Prothese in der Hülle mindestens ein elastisch deformierbarer, poriger Formkörper vorgesehen ist.

Durch die Verwendung des porigen Formkörpers, der vorteilhaft entweder eine offenzellige, schwammartige Struktur aufweist oder aus einem gitterartigen Fadengerippe bzw. Fadengespinst besteht, wird das Gewicht der Prothese wesentlich herabgesetzt, da der Formkörper durch seine porige Struktur ein relativ geringes Gewicht besitzt. Durch die Struktur des Formkörpers kann sich die träge fließende Masse, die insbesondere aus geliertem oder teilweise vulkanisiertem Silikon-Kautschuk besteht, bei Körperbewegungen auf einer Teiltiefe in die Poren oder Zellen des Formkörpers hinein- und zurückbewegen, und der Formkörper kann sich durch die Bewegung der gelartigen Masse elastisch verformen, so daß sich die Prothese wie eine natürliche Brust verhält.

Die schwingende Bewegung kann dadurch begünstigt werden, daß zwischen der gelartigen Masse und dem Formkörper ein Hohlraum vorgesehen ist, der die freie Bewegung der Masse begünstigt.

Der poröse Formkörper kann auch aus zwei oder mehreren Körpern bestehen oder aus mehreren Teilen, z. B. schichtförmig, zusammengesetzt sein. Durch die nur teilweise vulkanisierte bzw. gelierte Masse, insbesondere aus Silikon-Kautschuk, wird die Masse auf dem Umfang des Formkörpers in die Poren eindringen und aus dem ursprünglich flüssigen Zustand je nach Einstellung der Zusammensetzung mehr oder weniger stark gelieren bzw. vulkanisieren, so daß diese Masse praktisch mit dem Formkörper verbunden ist und diesen Formkörper in seiner in die Hülle eingebrachten Lage im wesentlichen festlegt.

Bei einer besonders vorteilhaften Ausführung ist der Formkörper im Bereich der dem Brustkorb zugekehrten Wandung der Hülle angeordnet, so daß der sonstige Teil unter Deformation des Formkörpers beweglich bleibt und schwingen kann.

Die Erfindung wird anhand von zwei Ausführungsbeispielen näher erläutert. Es zeigen:

Figur 1　　einen senkrechten Schnitt durch eine Brustprothese nach einem ersten Beispiel,

Figur 2　　einen Schnitt nach der Linie II-II der Figur 1,

Figur 3　　einen senkrechten Schnitt durch eine Brustprothese nach einem weiteren Ausführungsbeispiel und

Figur 4　　einen horizontalen Schnitt durch den oberen Bereich der Prothese nach Figur 1.

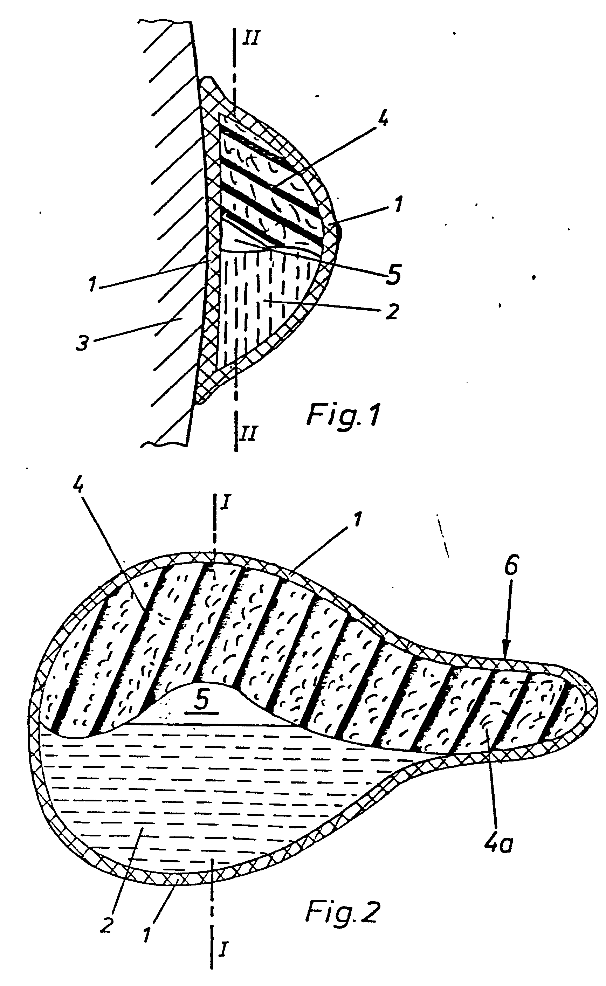

Nach den Figuren 1 und 2 besteht die Prothese aus einer geschlossenen, der natürlichen Brust nachgebildeten Hülle 1, die z. B. in bekannter Weise aus elastischem, voll vulkanisiertem Silikon-Kautschuk oder aus einem gleichwirkenden, elastischen Material hergestellt ist. Diese Hülle 1 ist teilweise mit einer träge fließenden, gelartigen Masse, insbesondere mit einem nur teilweise vulkanisierten bzw. gelierten und daher fließenden, gelartigen Silikon-Kautschuk 2 gefüllt, wobei dieser träge fließende Füllungsteil 2 beim Tragen der Prothese auf dem Körper 3 den unteren Teil des Hülleninnenraumes einnimmt. Der restliche obere Innenraum der Hülle 1 wird von einem elastisch deformierbaren Formkörper 4 von offenzelliger bzw. durchgehend offenporiger Struktur eingenommen, die der eines natürlichen oder künstlichen Schwammes entspricht und aus einem geeigneten Kunststoff oder Kautschuk besteht.

Vorteilhaft besitzt dieser Formkörper 4 die Struktur eines gitterartigen Fadengerippes oder eines Fadengespinstes aus Kautschuk, Silikon-Kautschuk oder einem Kunststoff, wie z. B. PVC, Polyamid, Polyurethan oder dergleichen.

Durch den offenzelligen Formkörper 4 kann das Gewicht der Prothese äußerst niedrig gehalten werden, so daß die Prothese von den Frauen nicht mehr als lästig empfunden wird, und vor allen Dingen wird die Bewegung der träge fließenden Masse 2 bei Körperbewegungen nicht behindert, denn die Masse kann bei Bewegungen des Frauenkörpers den Formkörper 4 elastisch verformen und wenigstens auf eine Teiltiefe in den Formkörper 4 eindringen und zurückfließen, so daß die Prothese sich bei Bewegung der Frau wie eine natürliche Brust verhält. Diese Bewegung der Füllungsmasse 2 kann dadurch begünstigt werden, daß zwischen der gelartigen Masse 2 und dem Formkörper 4 ein Hohlraum 5 vorgesehen wird, in den die Masse 2 einfließen und dann zurückfließen kann.

Es ist bekannt, eine der Brust nachgebildeten Prothese mit einem seitlichen, lappenartigen Fortsatz zu versehen, der im allgemeinen durch einen Büstenhalter zwangsweise gegen die seitliche Brustkorbfläche zur Anlage gebracht wird, um z. B. den Achsel-Lympfdrüsenbereich abzudecken.

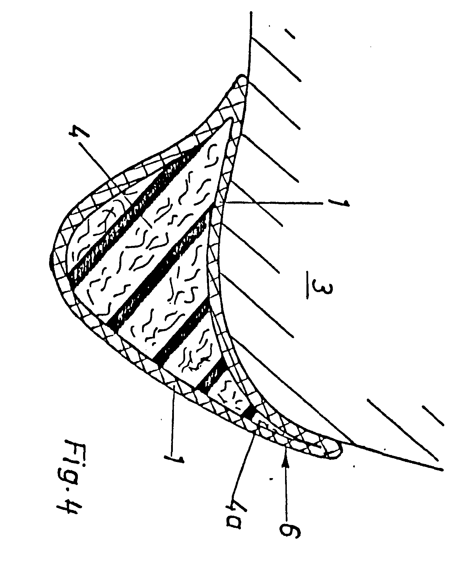

Demgegenüber wird nach der Erfindung so vorgegangen, daß die Hülle 1 der Prothese auf einer Seite den lappenartigen Fortsatz 6 bildet, der mit einem Fortsatz 4a des Formkörpers 4 ausgefüllt wird. Der Formkörper 4 mit dem Fortsatz 4a und die geformte Hülle 1 bringen die Prothese nach der Erfindung in eine Lage, in der die Prothese ein bogenförmig gekrümmtes, formstabiles, elastisches Gebilde ist, dessen an den Brustkorb 3 anliegende Fläche im wesentlichen konkav ist. Es ist damit von vornherein eine Anpassung der Prothese an die Form des Brustkorbes erreicht, und es ist nicht mehr erforderlich, daß ein Büstenhalter diese anliegende Form erzwingt. Die zugehörige Ausführungsform ist in Figur 4 dargestellt.

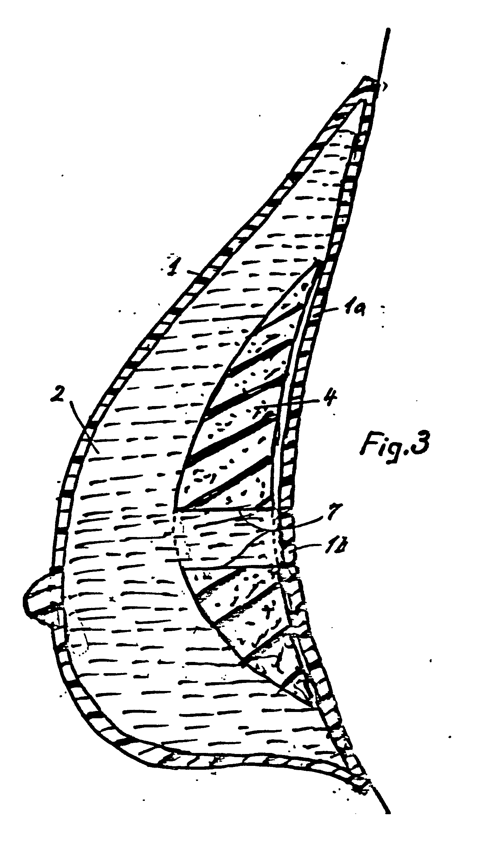

Nach dem besonders vorteilhaften Beispiel gemäß Figur 3, in der die gleichen Bezugszeichen der Figuren 1 und 2 für gleiche Teile verwendet sind, ist ein elastisch verformbarer, poröser Formkörper 4 oder es sind mehrere Formkörper in die elastische Hülle eingebracht. Der Formkörper 4, der auch aus mehreren Teilen oder Schichten aufgebaut sein kann, befindet sich vorteilhaft im Bereich der dem Brustkorb zugekehrten Hüllenwandung 1a, so daß die bewegliche gelartige Masse 2 den äußeren Innenraum der Hülle 1 einnimmt, wobei die schwingende Bewegung der Brust beim Gehen der Trägerin der schwingenden Bewegung einer natürlichen Brust besonders günstig nachgeahmt wird.

In diesem Fall soll die fließende Masse 2 nach dem Einfüllen in die Hülle 1 je nach Zusammensetzung in die Poren auf dem Umfang des Formkörpers 4 mehr oder weniger stark eindringen und einen gelartigen bzw. teilweise vulkanisierten Zustand einnehmen, so daß dadurch die Masse 2 den Formkörper 4 in seiner Lage im wesentlichen in der Hülle festlegt.

In diesem Fall ist der Formkörper 4 für das Einfüllen der träge fließenden Masse 2 mit einer kanalförmigen Durchbrechung 7 versehen, und es muß die Hüllenwandung 1a einen mit dem Kanal fluchtenden Durchgang 1b aufweisen, so daß das Einfüllen der gelartigen Masse 2 in die Hülle 1 durch diese Durchbrechungen erfolgen kann. Die Hüllendurchbrechung 1b wird anschließend wieder verschlossen.

Anstelle einer gelartigen Masse aus Silikon-Kautschuk können auch andere Massen, wie Öle, Pasten und dergleichen, als Füllmaterial verwendet werden. Unter dem Begriff "träge fließende Masse" ist also nicht unbedingt eine flüssige Masse zu verstehen. Beispielsweise sind gelartige Massen oder teilweise kalt vulkanisierte Massen in diesem Sinne "träge fließend", wenn sie sich zwar durch Einwirkung einer Kraft fließend elastisch verformen lassen, aber nach Aufhebung der Kraft im wesentlichen wieder ihre ursprüngliche Form annehmen.

**Patentansprüche**

1. Prothese als Ersatz für eine amputierte Brust, bestehend aus einer der Brustform nachgebildeten Hülle (1) aus elastischem Material mit einer Füllung aus einer träge fließenden Masse (2), dadurch gekennzeichnet, daß in der Hülle (1) mindestens ein elastisch deformierbarer, poriger Formkörper (4) vorgesehen ist.

2. Prothese nach Anspruch 1, dadurch gekennzeichnet, daß der Formkörper (4) eine offenzellige schwammartige Struktur aufweist.

3. Prothese nach Anspruch 1, dadurch gekennzeichnet, daß der Formkörper (4) aus einem gitterartigen Fadengeripps bzw. Fadengespinst besteht.

4. Prothese nach einem der Ansprüche 1 bis 3, dadurch gekennzeichnet, daß der Formkörper (4) durch die gelartige Masse (2), wie Silikon-Kautschuk, in seiner Lage in der Hülle (1) im wesentlichen festgelegt ist.

5. Prothese nach Anspruch 4, dadurch gekennzeichnet, daß die Masse (2) auf dem Umfang des Formkörpers (4) in dessen Poren bleibend eingedrungen ist.

6. Prothese nach einem der Ansprüche 1 bis 5, dadurch gekennzeichnet, daß der Formkörper (4) im Bereich der dem Brustkorb zugekehrten Hüllenwandung (1a) angeordnet ist.

7. Prothese nach einem der Ansprüche 1 bis 6, dadurch gekennzeichnet, daß der Formkörper (4) im Mittelteil einen freien Durchgang (7) besitzt, der mit einem verschlossenen Durchgang (1b) der hinteren Hüllenwandung fluchtet.

8. Prothese nach einem der Ansprüche 1 bis 7, dadurch gekennzeichnet, daß zwischen der Masse (2) und dem im oberen Teil der Hülle befindlichen Formkörper (4) ein Hohlraum (5) vorgesehen ist.

9. Prothese nach einem der Ansprüche 1 bis 8, dadurch gekennzeichnet, daß sie zur Armseite mit einem lappenartigen Fortsatz (6) versehen ist und daß sie mit dem Fortsatz (6) ein formstabiles, bogenförmig gekrümmtes, elastisch verformbares Gebilde ist, dessen an den Brustkorb anliegende Hüllenfläche (1a) im wesentlichen konkav ist.

## Claims

1. Prosthesis as a substitute for an amputated breast comprising an envelope (1) of elastic material formed in imitation of the shape of a breast, with a filling of a slow-flowing mass (2), characterised in that at least one elastically deformable porous shaped element (4) is provided within the envelope (1).

2. A prosthesis according to claim 1, characterised in that the shaped element (4) has an open-celled sponge-like structure.

3. A prosthesis according to claim 1, characterised in that the shaped element (4) consists of a grid-like thread framework or thread web.

4. A prosthesis according to one of claims 1 to 3, characterised in that the shaped element (4) is substantially immobilised in its position within the envelope (1) by means of the gel-like mass (2), such as silicone rubber.

5. A prosthesis according to claim 4, characterised in that the mass (2) has penetrated permanently into the pores of the shaped element (4) over its periphery.

6. A prosthesis according to one of claims 1 to 5, characterised in that the shaped element (4) is situated in the area of the envelope wall (1a) facing towards the ribcage.

7. A prosthesis according to one of claims 1 to 6, characterised in that the shaped element (4) has an open passage (7) in the central portion, which is aligned with a closed passage (16) of the rear wall of the envelope.

8. A prosthesis according to one of claims 1 to 7, characterised in that a cavity (5) is provided between the mass (2) and the moulded element (4) situated in the upper part of the envelope.

9. Prosthesis according to one of claims 1 to 8, characterised in that it is provided with a flap-like extension (6) towards the side of the arm and that together with extension (6) it is a morphologically stable arcuately curved and elastically deformable structure of which the envelope surface (1a) in contact with the ribcage is substantially concave.

## Revendications

1. Prothèse servant de substitut pour un sein amputé, constituée par une enveloppe (1) imitant la forme du sein et réalisée dans une matière élastique, avec un remplissage d'une masse visqueuse (2), <u>caractérisée en ce</u> qu'au moins un corps préformé (4) poreux et élastiquement déformable est prévu dans l'enveloppe (1).

2. Prothèse selon la revendication 1, caractérisée en ce que le corps préformé (4) présente une structure d'éponge à alvéoles ouvertes.

3. Prothèse selon la revendication 1, caractérisée en ce que le corpspréformé (4) est constitué par une carcasse ou un filet de fil en forme de treillis.

4. Prothèse selon l'une des revendications 1 à 3, caractérisée en ce que la position du corpspréformé (4) à l'intérieur de l'enveloppe (1) est sensiblement définie par la masse (2) semblable à du gel telle que du caoutchouc silicone.

5. Prothèse selon la revendication 4, caractérisée en ce que la masse (2) a pénétré de manière durable dans les pores à la périphérie du corps préformé (4).

6. Prothèse selon l'une des revendications 1 à 5, caractérisée en ce que le corps préformé (4) est disposé dans la région de la paroi de l'enveloppe (1a) dirigée vers la cage thoracique.

7. Prothèse selon l'une des revendications 1 à 6, caractérisée en ce que le corps préformé (4) présente dans sa partie centrale un passage libre (7) qui est aligné avec un passage fermé (1b) de la paroi arrière de l'enveloppe.

8. Prothèse selon l'une des revendications 1 à 7, caractérisée en ce qu'une cavité (5) est prévue entre la masse (2) et le corps préformé (4) placé dans la partie supérieure de l'enveloppe.

9. Prothèse selon l'une des revendications 1 à 8, caractérisé en ce qu'elle est munie, en direction du bras, d'un appendice (6) en forme de languette et qu'elle constitue conjointement avec l'appendice (6) une structure de forme stable, courbée en arc et élastiquement déformable dont la surface d'enveloppe (1a) appliquée contre la cage thoracique est sensiblement concave.

Fig.1

Fig.2

*Fig.3*

Fig. 4